# FASCICULE DE BREVET EUROPEEN

(11) **EP 3 010 592 B1**
(45) Date de publication et mention de la délivrance du brevet: **17.10.2018**
(21) Numéro de dépôt: 14730897.7
(22) Date de dépôt: 17.06.2014
(51) Int. Cl.: A61Q 5/06, A61K 8/31, A61K 8/37, A61K 8/39, A61K 8/81, A61K 8/36, A61K 8/92, A61K 8/86

(54) **COMPOSITION COSMETIQUE COMPRENANT UNE CIRE MINERALE, UN ACIDE GRAS, UNE HUILE MINERALE, UN TENSIOACTIF, UN ESTER D'ACIDE GRAS ET/OU D'ALCOOL GRAS, UN POLYMERE FIXANT**
KOSMETISCHE ZUSAMMENSETZUNG MIT EINEM MINERALISCHEN WACHS, EINER FETTSÄURE, EINEM MINERALÖL, EINEM TENSID, EINER FETTSÄURE UND/ODER EINEM FETTALKOHOLESTER UND EINEM FIXIERENDEN POLYMER
COSMETIC COMPOSITION INCLUDING A MINERAL WAX, A FATTY ACID, A MINERAL OIL, A SURFACTANT, A FATTY ACID AND/OR FATTY ALCOHOL ESTER, AND A FIXING POLYMER

(30) Priorité: 17.06.2013 FR 1355636; 17.06.2013 FR 1355638; 17.06.2013 FR 1355637
(43) Date de publication de la demande: 27.04.2016
(73) Titulaire: L'OREAL, 75008 Paris (FR)
(72) Inventeur: FONDIN, Thomas, F-95150 Taverny (FR); CLEMENT, Franck, F-91700 Sainte Genevieve Des Bois (FR)
(74) Mandataire: Leray, Noelle
(86) Numéro de dépôt international: PCT/EP2014/062634
(87) Numéro de publication internationale: WO 2014/202559

(56) Documents cités:
- WO-A1-2011/076566
- DE-A1-102007 052 391
- DATABASE GNPD [Online] MINTEL; août 2011 (2011-08), "Thousle Whip 04 Cream-Wax", XP002720285, Database accession no. 1621961
- DATABASE GNPD [Online] MINTEL; octobre 2012 (2012-10), "5 Design Cream", XP002720286, Database accession no. 1973765
- DATABASE GNPD [Online] MINTEL; septembre 2010 (2010-09), "Elastic Hair Styling Wax", XP002720287, Database accession no. 1387310
- DATABASE GNPD [Online] MINTEL; juin 2011 (2011-06), "Wax", XP002720288, Database accession no. 1577698
- DATABASE GNPD [Online] MINTEL; septembre 2012 (2012-09), "Clay Strong Hair Wax", XP002720527, Database accession no. 1895177

## Description

La présente invention concerne une composition cosmétique comprenant au moins une cire minérale, au moins un acide gras, au moins une huile minérale, au moins un tensioactif, au moins un ester d'acide gras et/ou d'alcool gras et au moins un polymère fixant, ainsi que l'utilisation d'une telle composition pour le traitement capillaire, notamment pour le traitement des fibres kératiniques et en particulier pour le maintien/la mise en forme des cheveux.

Les produits de coiffage à effet cire se présentent majoritairement sous forme de pâtes plus ou moins visqueuses qui s'appliquent sur les cheveux avec les mains.

Or, les cires de coiffage sont souvent collantes et grasses. En outre, la coiffure obtenue est difficilement repositionnable et présente un rendu collant.

Il existe donc un réel besoin de disposer d'une composition cosmétique qui présente de bonnes propriétés coiffantes et cosmétiques, et qui permette de remédier aux inconvénients cités ci-dessus.

Le document DE 10 2007 052 391 décrit une composition cosmétique pour la mise en forme temporaire des fibres kératiniques qui comprend une cire et une poudre composite. Ce document ne décrit pas une composition comprenant entre autres de 12 à 40 % en poids d'une cire minérale.

La Demanderesse a découvert qu'en associant au moins une cire minérale dans une teneur particulière, au moins un acide gras, au moins une huile minérale, au moins un tensioactif et au moins un ester d'acide gras et/ou d'alcool gras, il était possible d'obtenir des cires de coiffage avec des qualités d'usage améliorées et des performances de coiffage améliorées.

La présente invention a donc pour objet une composition cosmétique, comprenant (i) au moins une cire minérale dans une teneur allant de 12 à 40% en poids par rapport au poids total de la composition, (ii) au moins un acide gras, (iii) au moins une huile minérale, (iv) au moins un tensioactif, (v) au moins un ester d'acide gras et/ou d'alcool gras et au moins un polymère fixant.

La composition cosmétique est de préférence une composition de coiffage et/ou de conditionnement des fibres kératiniques, notamment de coiffage des fibres kératiniques, en particulier des fibres kératiniques humaines telles que les cheveux.

L'invention concerne également un procédé de traitement cosmétique des fibres kératiniques, notamment pour le maintien et/ou la mise en forme des fibres kératiniques, mettant en oeuvre la composition cosmétique telle que définie ci-avant.

L'invention a encore pour objet l'utilisation d'une composition telle que définie précédemment pour le traitement capillaire, notamment pour le traitement des fibres kératiniques et en particulier pour le maintien et/ou la mise en forme des cheveux.

La composition obtenue est facile à répartir dans les mains puis sur les cheveux. En outre, la coiffure est rapide à mettre en forme. Les cheveux sont peu collants et on obtient une coiffure au rendu naturel. La tenue de la coiffure est améliorée et le recoiffage est facilité.

D'autres objets, caractéristiques, aspects et avantages de l'invention apparaîtront encore plus clairement à la lecture de la description et des exemples qui suivent.

Dans ce qui suit l'expression « au moins un » est équivalente à l'expression « un ou plusieurs ».

La composition selon l'invention comprend au moins une **cire minérale.**

Les cires considérées dans le cadre de la présente invention sont d'une manière générale des composés lipophiles, solides, déformables ou non déformables, à température ambiante (25 °C), à changement d'état solide/liquide réversible, ayant un point de fusion supérieur ou égal à 30 °C pouvant aller jusqu'à 200 °C et notamment jusqu'à 120 °C.

En portant une ou des cires, conforme(s) à l'invention, à l'état liquide (fusion), il est possible de la ou les rendre miscibles à une ou plusieurs huiles et de former un mélange cire(s) + huile(s), homogène macroscopiquement, mais en ramenant la température dudit mélange à la température ambiante, on obtient une recristallisation de la ou des cire(s) dans la ou les huile(s) du mélange.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire, disposé dans un creuset, est soumis à une première montée en température allant de - 20 °C à 100 °C, à la vitesse de chauffe de 10 °C/minute, puis est refroidi de 100 °C à - 20 °C à une vitesse de refroidissement de 10 °C/minute et enfin est soumis à une deuxième montée en température allant de - 20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans une composition selon l'invention sont choisies parmi les cires, de préférence solides à température ambiante, d'origine minérale.

Au sens de la présente invention, par cire minérale on entend une cire issue du pétrole, telle que la cire de paraffine, l'ozokérite, la cérésine ou les cires microcristallines comme par exemple les cires microcristallines dont le point de fusion est supérieur à 85 °C telles que les produits HI-MIC® 1070, 1080, 1090 et 3080 commercialisés par la société NIPPON SEIRO.

Selon un mode de réalisation particulier, la cire utilisée dans une composition conforme à l'invention présente un point de fusion supérieur à 35 °C, mieux supérieur à 40 °C voire à 45 °C, ou encore à 55 °C.

Selon un mode de réalisation particulier de l'invention, la composition comprend une cire microcristalline et/ou l'ozokérite.

La teneur en cire(s) minérale(s) varie de 12 à 40 %, de préférence de 12 à 35 %, mieux de 12 à 30 % en poids par rapport au poids total de la composition.

La composition selon l'invention comprend également au moins un **acide gras.**

L'acide gras peut être liquide ou non liquide.

Par acide gras liquide, on entend un acide gras liquide à température ordinaire (25 °C) et à pression atmosphérique (760 mm de Hg, soit 1,013.10⁵ Pa).

De préférence, les acides gras de l'invention comportent de 8 à 30 atomes de carbone.

Les acides gras de l'invention peuvent être saturés ou insaturés.

Les acides gras liquides saturés sont de préférence ramifiés. Ils peuvent éventuellement comprendre dans leur structure au moins un cycle aromatique ou non. De préférence, ils sont acycliques.

On peut citer plus particulièrement l'acide isostéarique.

Les acides gras insaturés présentent dans leur structure au moins une double ou triple liaison, et de préférence, une ou plusieurs doubles liaisons. Lorsque plusieurs doubles liaisons sont présentes, elles sont de préférence au nombre de 2 ou 3 et elles peuvent être ou non conjuguées.

Ces acides gras insaturés peuvent être linéaires ou ramifiés.

Ils peuvent éventuellement comprendre dans leur structure au moins un cycle aromatique ou non. De préférence, ils sont acycliques.

Dans une première variante les acides gras de l'invention sont liquides.

On peut citer plus particulièrement l'acide oléique.

Dans une seconde variante de l'invention, particulièrement appréciée, l'acide gras est non liquide et de préférence solide.

Les acides gras non liquides convenant à la mise en oeuvre de l'invention sont plus particulièrement choisis parmi les acides saturés ou insaturés, linéaires ou ramifiés, comportant de 8 à 30 atomes de carbone.

On citera par exemple comme acide gras de ce type, l'acide stéarique, l'acide palmitique, l'acide myristique, l'acide béhénique et leurs mélanges.

Le ou les acides gras peuvent être présents dans la composition dans une teneur allant de 0,1 à 10%, de préférence de 0,5 à 7%, mieux de 1 à 5%, en poids par rapport au poids total de la composition.

La composition selon la présente invention comprend également une ou plusieurs **huiles minérales.**

On entend par "huile", tout composé lipophile, non ionique, insoluble dans l'eau et liquide à température ambiante (25 °C) et sous pression atmosphérique. Par insoluble dans l'eau, on entend au sens de la présente invention, un composé dont la solubilité à pH spontané dans l'eau à 25°C et à pression atmosphérique est inférieure à 1 % et de préférence inférieure à 0,5 %. Les huiles ont de préférence une température de fusion inférieure à 5°C et une viscosité inférieure à 500 cPs à 25 °C à un taux de cisaillement de 1s⁻¹.

On entend par "huiles minérales" des hydrocarbures sous forme d'huiles, linéaires ou ramifiés, saturés ou insaturés, d'origine minérale ou synthétique, et pouvant être hydrogénées.

Les huiles minérales utilisées dans la présente invention sont choisies parmi les huiles minérales, telles que définies ci-dessus, habituellement employées dans le domaine cosmétique.

A titre d'exemples d'huiles minérales utilisables dans la présente invention, on peut citer :
- les mélanges d'huiles hydrocarbonées dérivées du pétrole (nom INCI : Minerai Oil),
- l'huile de paraffine, volatile ou non volatile,
- l'huile de vaseline,
- les polyoléfines et en particulier les polydécènes,
- les isoparaffines telles que l'isohexadécane, l'isododécane et les polyisobutylènes hydrogénés tels que l'huile de Parléam® commercialisée par la société NOF Corporation (nom INCI : Hydrogenated Polyisobutene).

Parmi les huiles minérales citées ci-dessus, on utilise de préférence :
- les mélanges d'huiles hydrocarbonées dérivées du pétrole,
- l'huile de paraffine, volatile ou non volatile, et,
- l'huile de vaseline, et
- les polyoléfines et en particulier les polydécènes.

Par "polydécènes", on entend tous composés de formule C₁₀ₙH₍₂₀ₙ₎₊₂ avec n variant de 3 à 9, répondant à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes. Parmi ces composés, on choisit plus particulièrement selon l'invention ceux pour lesquels dans la formule, n varie de 3 à 7.

On peut citer, à titre d'exemple, et de préférence, le produit vendu sous la dénomination SILKFLO® 366 NF POLYDECENE par la société AMOCO CHEMICAL, ceux vendus sous la dénomination NEXBASE® 2002 FG, 2004 FG, 2006 FG et 2008 FG par la société FORTUM.

L'huile minérale préférée est l'huile de vaseline.

L'huile ou les huiles minérale(s) peuvent être présentes dans la composition dans une teneur allant de préférence de 0,1 à 10 % en poids, et mieux de 0,5 à 7 % en poids, et plus particulièrement de 1 à 5 % en poids du poids total de la composition finale.

La composition selon l'invention comprend également un ou plusieurs **esters d'alcool gras et/ou d'acide gras** et de préférence d'acide gras et d'alcool gras et plus particulièrement d'acide gras saturé et de mono-alcool gras saturé.

Par alcool gras ou acide gras, on comprend des composés comprenant au moins 10 atomes de carbone, de préférence de 10 à 50 atomes de carbone.

Les esters d'alcool gras et/ou d'acide gras peuvent être liquides ou solides à 25°C et à la pression atmosphérique (10⁵ Pa).

A titre d'esters gras liquides on peut citer les myristate et palmitate d'isopropyle.

De préférence les esters gras de l'invention sont solides à 25°C et à la pression atmosphérique (10⁵ Pa).Les esters gras utilisés dans la composition de l'invention sont de préférence des esters d'acide gras saturés, c'est-à-dire des esters d'acides carboxyliques saturés comportant au moins 10 atomes de carbone, et de monoalcools gras saturés comportant au moins 10 atomes de carbone. Les acides ou les monoalcools saturés peuvent être linéaires ou ramifiés. Les acides carboxyliques saturés comportent de préférence de 10 à 30 atomes de carbone et plus particulièrement de 12 à 24 atomes de carbone. Ils peuvent être éventuellement hydroxylés. Les monoalcools gras saturés comportent de préférence de 10 à 30 atomes de carbone et plus particulièrement de 12 à 24 atomes de carbone.

De préférence, les esters d'alcool gras et/ou d'acide gras sont choisis parmi les myristates de myristyle, de cétyle et de stéaryle, les palmitates de myristyle, de cétyle et de stéaryle, les stéarates de myristyle de cétyle et de stéaryle, le béhénate de béhényle et leurs mélanges.

Le ou les esters d'alcool gras et/ou d'acide gras peuvent être présents dans la composition dans une teneur allant de à 1% à 20% en poids, de préférence de 2% à 15% en poids et plus préférentiellement de 4 % à 10 % en poids par rapport au poids total de la composition.

La composition cosmétique selon l'invention comprend également un ou plusieurs **tensioactifs** pouvant être choisis parmi les tensioactifs anioniques, cationiques, non ioniques, amphotères ou zwittérioniques ainsi que leurs mélanges.

De préférence le ou les tensioactifs sont choisis parmi les tensioactifs non ioniques.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools gras polyéthoxylés, polypropoxylés ou polyglycérolés, les alpha-diols polyéthoxylés, polypropoxylés ou polyglycérolés, les alkyl(C₁₋₂₀)phénols polyéthoxylés, polypropoxylés ou polyglycérolés, la chaîne grasse comportant, par exemple, de 8 à 18 atomes de carbone, le nombre de groupements oxyde d'éthylène ou oxyde de propylène pouvant aller notamment de 1 à 150 et le nombre de groupements glycérol pouvant aller notamment de 1 à 30.

On peut également citer les condensats d'oxyde d'éthylène et d'oxyde de propylène sur des alcools gras; les amides gras polyéthoxylés ayant de préférence de 1 à 100 motifs d'oxyde d'éthylène, les amides gras polyglycérolés comportant en moyenne de 1 à 5 groupements glycérol et en particulier de 1,5 à 4, les esters d'acides gras et de sorbitane éthoxylés ayant de 1 à 50 motifs d'oxyde d'éthylène, les esters d'acides gras du saccharose, les esters d'acides gras et de polyéthylèneglycol, les alkylpolyglycosides, les huiles végétales polyéthoxylées ayant de préférence de 1 à 100 motifs d'oxyde d'éthylène, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Les alkylpolyglucosides peuvent être choisis par exemple parmi le décylglucoside (Alkyl-C₉/C₁₁-polyglucoside (1,4)) comme le produit commercialisé sous la dénomination Mydol 10® par la société Kao Chemicals ou le produit commercialisé sous la dénomination Plantacare 2000 UP® par la société Henkel et le produit commercialisé sous la dénomination ORAMIX NS 10® par la société SEPPIC ; le caprylyl/capryl glucoside comme le produit commercialisé sous la dénomination Plantacare KE 3711® par la société Cognis ou ORAMIX CG 110® par la société SEPPIC ; le laurylglucoside comme le produit commercialisé sous la dénomination Plantacare 1200 UP® par la société Henkel ou Plantaren 1200 N® par la société Henkel ; le cocoglucoside comme le produit commercialisé sous la dénomination Plantacare 818 UP® par la société Henkel ; le caprylylglucoside comme le produit commercialisé sous la dénomination Plantacare 810 UP® par la société Cognis ; et leurs mélanges.

De préférence, les tensioactifs sont choisis parmi les alcools gras oxyéthylénés.

Le ou les tensioactifs peuvent être présents dans une teneur allant de 0,1 à 30 % en poids, de préférence dans une teneur allant de 0,5 à 15 % en poids, par rapport au poids total de la composition.

Selon un mode de réalisation, la composition peut comprendre un ou plusieurs alcools gras oxyéthylénés ayant de 1 à 5 motif(s) d'oxyde d'éthylène.

Par alcool gras oxyéthyléné, on entend au sens de la présente invention un alcool oxyéthyléné ayant une chaîne hydrocarbonée comportant au moins 6 atomes de carbone.

Par alcool gras oxyéthyléné selon l'invention, on entend tout alcool gras de structure suivante : 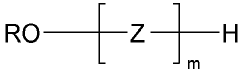 dans laquelle :
R désigne un radical saturé ou insaturé, linéaire ou ramifié, comportant de 6 à 40 atomes de carbone, en particulier de 8 à 30, de préférence de 10 à 20, et Z représente un radical oxyéthyléné de formule suivante : 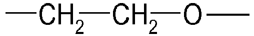 m représente le nombre de motifs d'oxyde d'éthylène allant de 1 à 5.

Des alcools gras oxyéthylénés liquides particulièrement préférés selon l'invention sont des alcools gras, saturés ou non, linéaires, comportant de 10 à 20 atomes de carbone, notamment 16 atomes de carbone, et de 2 à 5 motifs d'oxyde d'éthylène, en particulier deux motifs d'oxyde d'éthylène.

Comme composés de type alcool gras oxyéthyléné, on peut notamment citer les produits commercialisés suivants :
BRIJ S2-SO-(SG) (CRODA) [alcool stéarylique 2 OE] ;
Mergital LM2 (COGNIS) [alcool laurique 2 OE] ;
Empilan KA 2.5/90FL (ALBRIGHT & WILSON) et Mergital BL309 (COGNIS) [alcool décylique 3 OE] ;
Empilan KA 5/90 FL (ALBRIGHT & WILSON) et Mergital BL589 (COGNIS) [alcool décylique 5 OE] ;
Emulgin 05 (COGNIS) [alcool oléocétylique 5 OE].

De préférence, l'alcool gras oxyéthyléné présent dans la composition cosmétique selon l'invention est l'alcool stéarylique comprenant deux motifs d'oxyde d'éthylène.

Lorsqu'elle en comprend, la composition comprend un ou plusieurs alcools gras oxyéthylénés ayant de 1 à 5 motif(s) d'oxyde d'éthylène en une quantité allant de de 0,1 à 10 %, de préférence de 0,5 à 8 %, mieux de 2 à 6 %, en poids par rapport au poids total de la composition.

Selon un mode de réalisation, la composition cosmétique selon l'invention peut comprendre un ou plusieurs tensioactifs non ioniques oxyéthylénés à chaîne(s) grasse(s) insaturée(s) à au moins 10 motifs d'oxyde d'éthylène. A titre d'exemples de tensioactifs non ioniques oxyéthylénés à chaîne(s) grasse(s) insaturée(s), on peut citer :
les alkyl(C₈-C₂₄)phénols oxyéthylénés,
les alcools gras en C₈-C₃₀, de préférence en C₁₂-C₂₂, insaturés, linéaires ou ramifiés, oxyéthylénés,
les amides, en C₈-C₃₀, insaturés, linéaires ou ramifiés, oxyéthylénés,
les esters d'acides en C₈-C₃₀, insaturés, linéaires ou ramifiés, et de sorbitol polyoxyéthylénés,
les huiles végétales oxyéthylénées, insaturées,
et leurs mélanges.

De préférence, les tensioactifs non ioniques oxyéthylénés à chaîne(s) grasse(s) insaturée(s) sont choisis parmi les alcools gras insaturés oxyéthylénés.

Par alcool gras insaturés, on entend au sens de la présente invention un alcool comportant au moins six atomes de carbone et au moins une insaturation dans sa structure.

En particulier, les tensioactifs non ioniques oxyéthylénés à chaîne(s) grasse(s) insaturée(s) sont choisis parmi les alcools gras insaturés oxyéthylénés dont la chaîne grasse de l'alcool gras est en C₈-C₃₀, en particulier en C₁₀-C₂₂, insaturés, linéaires ou ramifiés, et ayant un nombre de motifs d'oxyde d'éthylène allant de 10 à 150 comme par exemple les produits d'addition d'oxyde d'éthylène avec l'alcool oléylique, comportant de 10 à 150 motifs d'oxyde d'éthylène (par exemple Oleth-10, Oleth-11, Oleth-12, Oleth-15, Oleth-16, Oleth-20, Oleth-23, Oleth-24, Oleth-25, Oleth-30, Oleth-35, Oleth-40, Oleth-44, Oleth-50, Oleth-82, Oleth-106 en noms CTFA) ; les produits d'addition d'oxyde d'éthylène avec l'alcool undecylénique comportant de 10 à 50 motifs d'oxyde d'éthylène (par exemple Undeceth-11) ; et leurs mélanges.

De préférence, les tensioactifs non ioniques oxyéthylénés à chaîne(s) grasse(s) insaturée(s) de l'invention ont un nombre de motifs d'oxyde éthylène allant de 10 à 50.

Encore plus préférentiellement les tensioactifs non ioniques oxyéthylénés à chaîne(s) grasse(s) insaturée(s) sont choisis parmi les alcools oléyliques avec un nombre de motifs d'oxyde éthylène allant de 10 à 50.

Lorsqu'elle en comprend, la composition comprend un ou plusieurs tensioactifs non ioniques oxyéthylénés à chaîne(s) grasse(s) insaturée(s) à au moins 10 motifs d'oxyde éthylène en une quantité allant de 0,5 % à 25% en poids, mieux de 1% à 20 % en poids, encore mieux de 2 à 10% en poids par rapport au poids total de la composition.

La composition selon l'invention comprend également un ou plusieurs **polymères fixants.**

Au sens de l'invention on entend par polymère fixant tout polymère susceptible par application sur les cheveux de conférer une forme à la chevelure ou de permettre le maintien d'une forme déjà acquise.

Le ou les polymères fixants utilisés sont choisis parmi les polymères fixants ioniques, notamment anioniques, cationiques, amphotères, et non ioniques, et leurs mélanges.

A titre de polymères anioniques, on peut citer les polymères comportant des groupes dérivés d'acides carboxylique, sulfonique ou phosphorique, et présentant une masse moléculaire en nombre comprise entre 500 et 5 000 000.

Les groupements carboxyliques sont apportés par des monomères monoacides ou diacides carboxyliques insaturés tels que ceux répondant à la formule : 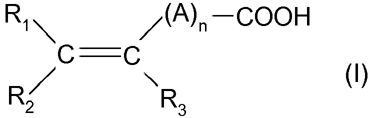 dans laquelle n est un nombre entier de 0 à 10, A désigne un groupement méthylène, éventuellement relié à l'atome de carbone du groupement insaturé ou au groupement méthylène voisin lorsque n est supérieur à 1, par l'intermédiaire d'un hétéroatome tel que l'oxygène ou le soufre, R₁ désigne un atome d'hydrogène, un groupement phényle ou benzyle, R₂ désigne un atome d'hydrogène, un groupement alkyle comportant de 1 à 4 atomes de carbone ou carboxyle, R₃ désigne un atome d'hydrogène, un groupement alkyle comportant de 1 à 4 atomes de carbone, un groupement -CH₂-COOH, phényle ou benzyle.

Dans la formule (I) ci-dessus, le groupement alkyle comportant de 1 à 4 atomes de carbone, peut désigner en particulier les groupements méthyle et éthyle.

Les polymères fixants anioniques à groupements carboxyliques ou sulfoniques préférés sont :
A) les copolymères d'acide acrylique ou méthacrylique ou leurs sels et d'acrylamide.
B) Les copolymères des acides acrylique ou méthacrylique avec un monomère monoéthylénique tel que l'éthylène, le styrène, les esters vinyliques, les esters d'acide acrylique ou méthacrylique éventuellement hydroxylés, éventuellement greffés sur un polyalkylène-glycol tel que le polyéthylène-glycol, et éventuellement réticulés. De tels polymères sont décrits en particulier dans le brevet français 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevets luxembourgeois 75370 et 75371. On peut également citer les copolymères d'acide acrylique et de méthacrylate d'alkyle en C₁-C₄, les copolymères acide méthacrylique/acide acrylique/acrylate d'éthyle/méthacrylate de méthyle, en particulier l'AMERHOLD DR 25 commercialisé par la société AMERCHOL les copolymères acide méthacrylique/acrylate d'éthyle, notamment en dispersion aqueuse, tels que les LUVIFLEX SOFT et LUVIMER MAE commercialisés par la société BASF, et les copolymères à base des hydroxyesters tels que l'ACUDYNE 180 dela société ROHM et HAAS,.
   Comme autre polymère fixant anionique de cette famille, on peut aussi citer le polymère anionique séquencé branché acrylate de butyle/acide acrylique/acide méthacrylique vendu sous la dénomination Fixate G-100 L par la société LUBRIZOL (nom INCI AMP-ACYLATES/ALLYL METHACRYLATE COPOLYMER).
C) les copolymères dérivés d'acide crotonique tels que ceux comportant dans leur chaîne des motifs acétate ou propionate de vinyle, et éventuellement d'autres monomères tels que les ester allylique ou méthallylique, éther vinylique ou ester vinylique d'un acide carboxylique saturé, linéaire ou ramifié, à longue chaîne hydrocarbonée, tels que ceux comportant au moins 5 atomes de carbone, ces polymères pouvant éventuellement être greffés et réticulés, ou encore un ester vinylique, allylique ou méthallylique d'un acide carboxylique α ou β-cyclique. De tels polymères sont décrits entre autres dans les brevets français numéros 1 222 944, 1 580 545, 2 265 782, 2 265 781, 1 564 110 et 2 439 798. Des produits commerciaux entrant dans cette classe sont les résines 28-29-30, 26-13-14 et 28-13-10 vendues par la société NATIONAL STARCH.
   On peut citer aussi comme copolymère dérivé d'acide crotonique les terpolymères acide crotonique/acétate de vinyle/ tertiobutylbenzoate de vinyle et en particulier le MEXOMERE PW fourni par la société CHIMEX.
D) Les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters ; ces polymères peuvent être estérifiés. De tels polymères sont décrits en particulier dans les brevets US 2 047 398, 2 723 248, 2 102 113, le brevet GB 839 805, et notamment ceux vendus sous les dénominations GANTREZ® AN ou ES par la société ISP.
   Des polymères entrant également dans cette classe sont les copolymères d'anhydrides maléique, citraconique, itaconique et d'un ester allylique ou méthallylique comportant éventuellement un groupement acrylamide, méthacrylamide, une α-oléfine, des esters acryliques ou méthacryliques, des acides acryliques ou méthacryliques ou la vinylpyrrolidone dans leur chaîne, les fonctions anhydrides sont monoestérifiées ou monoamidifiées. Ces polymères sont par exemple décrits dans les brevets français 2 350 384 et 2 357 241 de la demanderesse.
E) Les polyacrylamides comportant des groupements carboxylates.
F) Les polymères comprenant les groupements sulfoniques. Ces polymères peuvent être des polymères comportant des motifs vinylsulfonique, styrène-sulfonique, naphtalène-sulfonique, acrylamido-alkylsulfonique, sulfoisophtalates.
   Ces polymères peuvent être notamment choisis parmi :
   - les sels de l'acide polyvinylsulfonique ayant une masse moléculaire comprise entre environ 1 000 et 100 000 ainsi que les copolymères avec un comonomère insaturé tel que les acides acrylique ou méthacrylique, et leurs esters, ainsi que l'acrylamide ou ses dérivés, les éthers vinyliques et la vinylpyrrolidone ;
   - les sels de l'acide polystyrène-sulfonique, les sels de sodium, ayant une masse moléculaire d'environ 500 000 et d'environ 100 000. Ces composés sont décrits dans le brevet FR 2198719 ;
   - les sels d'acides polyacrylamide-sulfoniques tels que ceux mentionnés dans le brevet US 4 128 631 ;
G) les polymères siliconés anioniques greffés ;
   Les polymères siliconés greffés utilisés sont choisis préférentiellement parmi les polymères à squelette organique non-siliconé greffé par des monomères contenant un polysiloxane, les polymères à squelette polysiloxanique greffé par des monomères organiques non-siliconés et leurs mélanges.
H) Les polyuréthanes anioniques, pouvant comporter des greffons silicones et des silicones à greffons hydrocarbonés.

A titre d'exemples de polyuréthane fixant, on peut notamment citer le copolymère acide diméthylolpropionique/isophorone-diisocyanate/néopentylglycol/polyesterdiols (connu aussi sous le nom de polyuréthane-1, appellation INCI) vendu sous la marque Luviset® PUR par la société BASF, le copolymère acide diméthylol-propionique/isophorone-diisocyanate/néopentylglycol/ polyesterdiols/ diamine siliconée (connu aussi sous le nom de polyuréthane-6, appellation INCI) vendu sous la marque Luviset® Si PUR A par la société BASF.

Comme autre polyuréthane anionique, on peut aussi utiliser l'AVALURE UR 450.

On peut également utiliser les polymères à groupements sulfoisophtalates, tels que les polymères AQ55 et AQ48 commercialisés par la société EASTMAN.

Selon l'invention, les polymères anioniques sont de préférence choisis parmi les copolymères d'acide acrylique tels que le terpolymère acide acrylique/acrylate d'éthyle/N-tertiobutylacrylamide vendu sous la dénomination ULTRAHOLD STRONG® par la société BASF, les copolymères acide méthacrylique/acrylate d'éthyle, notamment en dispersion aqueuse, tels que les LUVIFLEX SOFT et LUVIMER MAE commercialisés par la société BASF. Des copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléïque monoestérifié vendu sous la dénomination GANTREZ® ES 425 par la société ISP, le LUVISET SI PUR, le MEXOMERE PW, les polyuréthanes anioniques élastomères ou non, les polymères à groupements sulfoisophtalates

Les polymères fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs de formules suivantes : 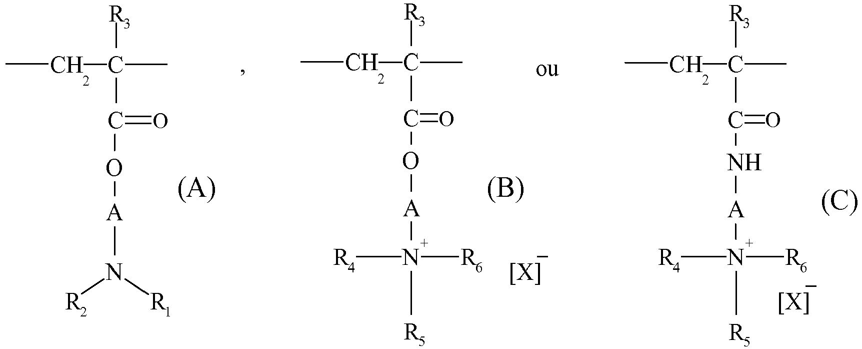 dans lesquelles :
   R₃ désigne un atome d'hydrogène ou un groupe CH₃;
   A est un groupe alkyle linéaire ou ramifié comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle;
   R₁ et R₂, identiques ou différents, représentent chacun un atome d'hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles en C₁-C₄, des groupes dérivés des acides acryliques ou méthacryliques ou leurs esters, des vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthyl-ammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyl-triméthylammonium tel que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT®" par la société ISP comme par exemple "GAFQUAT®734" ou "GAFQUAT®755" ou bien les produits dénommés "COPOLYMER® 845, 958 et 937". Ces polymères sont décrits en détail dans les brevets français 2.077.143 et 2.393.573,
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinylcaprolactame/vinylpyrrolidone tel que le produit commercialisé sous la dénomination GAFFIX VC 713 par la société ISP, et
   - et les copolymères vinylpyrrolidone/méthacrylamide de diméthylaminopropyle quaternisés tels que les produits commercialisés sous la dénomination "GAFQUAT® HS 100" par la société ISP.
(2) les gommes de guar cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane.
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL.
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes ; B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus que l'on préfère plus particulièrement, sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkyl-méthacrylamide et acrylamide. De tels composés sont décrits dans le brevet américain n° 3 836 537.
   Le composé vinylique peut être également un sel de dialkyldiallylammonium tel que le chlorure de diéthyldiallyl-ammonium.
2) les polymères comportant des motifs dérivant :
   a) d'au moins un monomère choisi parmi les acrylamides ou les méthacrylamides substitués sur l'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

   Les acrylamides ou méthacrylamides N-substitués plus particulièrement préférés selon l'invention sont les groupements dont les groupes alkyle contiennent de 2 à 12 atomes de carbone et plus particulièrement le N-éthylacrylamide, le N-tertiobutylacrylamide, le N-tertiooctylacrylamide, le N-octylacrylamide. le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butylaminoéthyle, de N,N'-diméthylaminoéthyle, de N-tertio-butylaminoéthyle. On utilise particulièrement les copolymères dont la dénomination CTFA (4ème Ed, 1991) est Octylacrylamide/ acrylates/butylaminoethylmethacrylate copolymer tels que les produits vendus sous la dénomination AMPHOMER® ou LOVOCRYL® 47 par la société NATIONAL STARCH.
3) les polyaminoamides réticulés et alkylés partiellement ou totalement dérivant de polyaminoamides de formule générale : 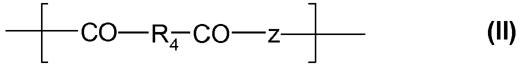 dans laquelle R₄ représente un groupe divalent dérivé d'un acide dicarboxylique saturé, d'un acide aliphatique mono ou dicarboxylique à double liaison éthylénique, d'un ester d'un alcool ayant 1 à 6 atomes de carbone de ces acides ou d'un groupe dérivant de l'addition de l'un quelconque desdits acides avec une amine bis primaire ou bis dérivé secondaire, et Z désigne un groupe d'une polyalkylène-polyamine bis-primaire, mono ou bis-secondaire et représente de préférence :
   a) dans les proportions de 60 à 100 % en moles, le groupe 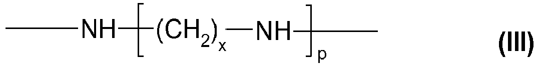
      où x=2 et p=2 ou 3, ou bien x=3 et p=2
      ce groupe dérivant de la diéthylène-triamine, de la triéthylène-tétraamine ou de la dipropylène-triamine;
   b) dans les proportions de 0 à 40 % en moles, le groupe (III) ci-dessus, dans lequel x=2 et p=1 et qui dérive de l'éthylènediamine, ou le groupe dérivant de la pipérazine 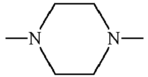
   c) dans les proportions de 0 à 20 % en moles, le groupe -NH-(CH2)6-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, 2,2,4-triméthyladipique et 2,4,4-triméthyladipique, téréphtalique, les acides à double liaison éthylénique comme, par exemple, les acides acrylique, méthacrylique, itaconique. Les alcane-sultones utilisées dans l'alcoylation sont de préférence la propane- ou la butane-sultone, les sels des agents d'alcoylation sont de préférence les sels de sodium ou de potassium.
4) les polymères comportant des motifs zwittérioniques de formule : 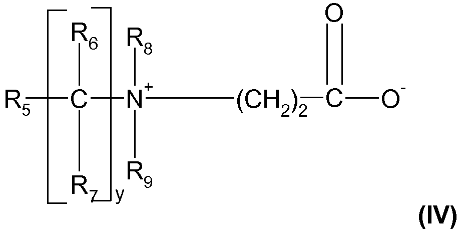 dans laquelle R₅ désigne un groupement insaturé polymérisable tel qu'un groupement acrylate, méthacrylate, acrylamide ou méthacrylamide, y et z représentent chacun un nombre entier de 1 à 3, R₆ et R₇ représentent un atome d'hydrogène, un groupement méthyle, éthyle ou propyle, R₈ et R₉ représentent un atome d'hydrogène ou un groupe alkyle de telle façon que la somme des atomes de carbone dans R₁₀ et R₁₁ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle, ou des acrylates ou méthacrylates d'alkyle, des acrylamides ou méthacrylamides, ou l'acétate de vinyle.
5) les polymères dérivés du chitosane comportant des motifs monomères répondant aux formules suivantes : 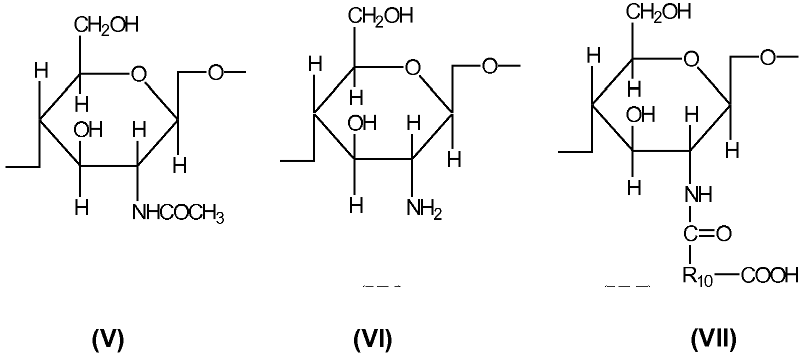 le motif (V) étant présent dans des proportions comprises entre 0 et 30 %, le motif (VI) dans des proportions comprises entre 5 et 50 % et le motif (VII) dans des proportions comprises entre 30 et 90 %, étant entendu que dans ce motif F, R₁₀ représente un groupe de formule: 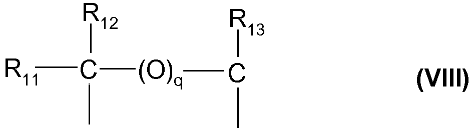
   dans laquelle si q=0, R₁₁, R₁₂ et R₁₃, identiques ou différents, représentent chacun un atome d'hydrogène, un reste méthyle, hydroxyle, acétoxy ou amino, un reste monoalkylamine ou un reste dialkylamine éventuellement interrompus par un ou plusieurs atomes d'azote et/ou éventuellement substitués par un ou plusieurs groupes amine, hydroxyle, carboxyle, alkylthio, sulfonique, un reste alkylthio dont le groupe alkyle porte un reste amino, l'un au moins des groupes R₁₇, R₁₈ et R₁₉ étant dans ce cas un atome d'hydrogène ;
   ou si q=1, R₁₁, R₁₂ et R₁₃ représentent chacun un atome d'hydrogène, ainsi que les sels formés par ces composés avec des bases ou des acides.
6) Les polymères dérivés de la N-carboxyalkylation du chitosane.
7) Les polymères de motifs répondant à la formule générale (IX) décrits par exemple, dans le brevet français 1 400 366 : 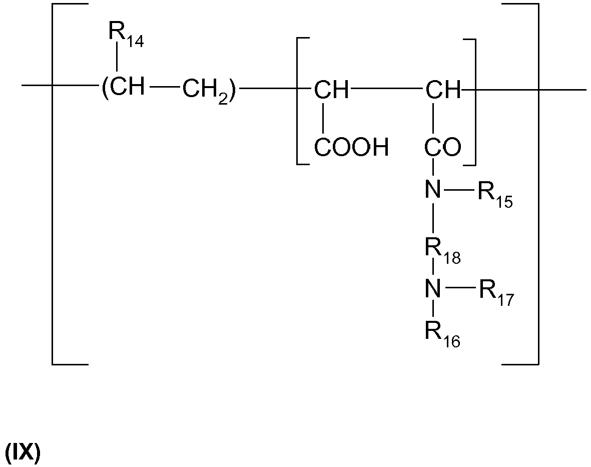
   dans laquelle R₁₄ représente un atome d'hydrogène, un groupe CH₃O, CH₃CH₂O, phényle, R₁₅ désigne l'hydrogène ou un groupe alkyle en C₁₋₄ tel que méthyle et éthyle, R₁₆ désigne l'hydrogène ou un groupe alkyle en C₁₋₄ tel que méthyle et éthyle, R₁₇ désigne un groupe alkyle en C₁₋₄ tel que méthyle et éthyle ou un groupe répondant à la formule: -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus,
   ainsi que les homologues supérieurs de ces groupes et contenant jusqu'à 6 atomes de carbone.
8) Des polymères amphotères du type -D-X-D-X- choisis parmi:
   a) les polymères obtenus par action de l'acide chloracétique ou le chloracétate de sodium sur les composés comportant au moins un motif de formule:

      -D-X-D-X-D- (X)

      où D désigne un groupe 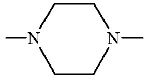 et X désigne le symbole E ou E', E ou E', identiques ou différents, désignent un groupe bivalent qui est un groupe alkylène à chaîne droite ou ramifiée comportant jusqu'à 7 atomes de carbone dans la chaîne principale non substituée ou substituée par des groupements hydroxyle, et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (XI)

      où D désigne un groupe 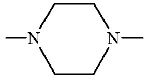 et X désigne le symbole E ou E' et au moins une fois E'; E ayant la signification indiquée ci-dessus et E' est un groupe bivalent qui est un groupe alkylène à chaîne droite ou ramifiée ayant jusqu'à 7 atomes de carbone dans la chaîne principale, substitué ou non par un ou plusieurs groupes hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
9) Les copolymères alkyl(C₁-C₅)vinyléther/anhydride maléique modifié partiellement par semiamidification avec une N,N-dialkylaminoalkylamine telle que la N,N-diméthylaminopropyl-amine ou par semiestérification avec une N,N-dialcanolamine Ces copolymères peuvent également comporter d'autres comonomères vinyliques tels que le vinylcaprolactame.

Selon un mode de réalisation préféré de l'invention, les polymères fixants amphotères utilisables dans le dispositif aérosol selon l'invention peuvent être choisis parmi les copolymères à blocs, ramifiés, comprenant :
(a) des motifs non ioniques dérivés d'au moins un monomère choisi parmi les (méth)acrylates d'alkyle en C₁-C₂₀, les N-mono-(alkyle en C₂-C₁₂)-(méth)acrylamides et les N,N-di-(alkyle en C₂-C₁₂)-(méth)acrylamide,
(b) des motifs anioniques dérivés d'au moins un monomère choisi parmi l'acide acrylique et l'acide méthacrylique, et
(c) des motifs polyfonctionnels dérivés d'au moins un monomère comportant au moins deux groupes fonctionnels insaturés polymérisables,
et ayant de préférence une structure constituée de blocs hydrophobes sur lesquels sont fixés, par l'intermédiaire des motifs polyfonctionnels (c), plusieurs blocs plus hydrophiles.

De préférence, les polymères amphotères présentent au moins deux températures de transition vitreuse (Tg) dont au moins une est supérieure à 20 °C et l'autre est inférieure à 20°C.

Les polymères amphotères préférés sont les polymères comportant des motifs dérivant :
a) d'au moins un monomère choisi parmi les acrylamides ou les méthacrylamides substitués sur l'azote par un groupe alkyle,
b) d'au moins un comonomère acide contenant un ou plusieurs groupements carboxyliques réactifs, et
c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou de diéthyle.

On peut citer, en particulier, les polymères vendus sous la dénomination AMPHOMER par la société NATIONAL STARCH.

Les polymères fixants non ioniques utilisables selon la présente invention sont choisis, par exemple, parmi :
- les polyalkyloxazolines,
- les homopolymères d'acétate de vinyle,
- les copolymères d'acétate de vinyle, tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle,
- les homopolymères et copolymères d'esters acryliques, tels que, par exemple, les copolymères d'acrylates d'alkyle et de méthacrylates d'alkyle, tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212,
- les copolymères d'acrylonitrile et d'un monomère non ionique choisis, par exemple, parmi le butadiène et les (méth)acrylates d'alkyle, tels que les produits proposés sous la dénomination CJ 0601 B par la société ROHM & HAAS,
- les homopolymères de styrène,
- les copolymères de styrène comme, par exemple, les copolymères de styrène et de (méth)acrylate d'alkyle, tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC, les copolymères de styrène, de méthacrylate d'alkyle et d'acrylate d'alkyle, les copolymères de styrène et de butadiène, ou les copolymères de styrène, de butadiène et de vinylpyridine,
- les polyamides,
- les homopolymères de vinyllactame, tels que les homopolymères de vinylpyrrolidone, le polyvinylcaprolactame commercialisé sous la dénomination Luviskol® PLUS par la société BASF,
- les copolymères de vinyllactame, tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF, et les terpolymères poly(vinylpyrrolidone/acétate de vinyle/propionate de vinyle) comme, par exemple, celui commercialisé sous la dénomination Luviskol® VAP 343 par la société BASF, et
- les poly(alcool vinylique).

Les groupes alkyles des polymères non ioniques mentionnés ci-dessus ont, de préférence, de 1 à 6 atomes de carbone.

De préférence, le polymère fixant est un polymère fixant non ionique ou cationique.

Encore plus préférentiellement, le polymère fixant est un polymère fixant non ionique.

Les polymères fixants peuvent être présents dans la composition en une quantité allant de 0,5 à 25%, de préférence de 0,7 à 20 %, de préférence encore de 1 à 17 % en poids par rapport au poids total de la composition.

De préférence, la composition comprend de l'eau, de préférence à une teneur supérieure ou égale à 5% en poids par rapport au poids total de la composition. La teneur en eau varie préférentiellement de 5 à 98%, de préférence de 10 à 95%, mieux de 20 à 80 %, encore mieux de 30 à 70% en poids par rapport au poids total de la composition.

La composition peut également comprendre un ou plusieurs solvants organiques liquides hydrosolubles choisis de préférence parmi les monoalcools tels que l'éthanol ou l'isopropanol; les polyols comme le propylèneglycol, le butylène glycol ou le glycérol ; les éthers de polyols ; et leurs mélanges.

La composition selon l'invention peut comprendre un agent propulseur. Par exemple, on peut citer les gaz liquéfiés comme le diméthyléther, le 1,1-difluoroéthane, ou les alcanes en C₃₋₅, comme le propane, l'isopropane, le n-butane, l'isobutane, le pentane, ou les gaz comprimés comme l'air, l'azote, le gaz carbonique, et leurs mélanges.

Préférentiellement, on utilisera les alcanes en C₃₋₅ et en particulier le propane, le n-butane, l'isobutane et leurs mélanges.

Lorsqu'elle en comprend, la composition comprend un ou des agents propulseur(s) en une quantité allant de 1 à 60 % en poids, mieux encore de 2 à 50 % en poids, et encore plus préférentiellement de 4 à 40 % en poids par rapport au poids total de la composition.

La composition selon l'invention peut contenir en outre un ou plusieurs additifs différent(s) des composés de l'invention choisi parmi les agents de conditionnement non siliconés, les silicones, les vitamines et pro-vitamines dont le panthénol, les filtres solaires, les agents nacrants et opacifiants, les colorants, les agents séquestrants, les agents épaississants, les agents plastifiants, les agents solubilisants, les agents acidifiants, les agents alcalinisants, les agents neutralisants, les agents anti-oxydants, les agents anti-mousse, les agents hydratants, les agents émollients, les hydroxyacides, les agents de pénétration, les parfums, les agents conservateurs et les charges et particules solides telles que par exemple les pigments minéraux et organiques, colorés ou non colorés.

Ces additifs peuvent être présents dans la composition selon l'invention en une quantité allant de 0 à 20 % en poids par rapport au poids total de la composition.

Bien entendu, l'homme du métier veillera à choisir les éventuels composés complémentaires, et/ou leur quantité, de manière telle que les propriétés avantageuses des compositions utilisées selon l'invention ne soient pas, ou substantiellement pas, altérées par l'adjonction envisagée.

La composition selon l'invention peut se présenter entre autres sous forme de liquides plus ou moins épaissis, de gels, de sérums, de crèmes, de pâtes, de sprays ou de mousses.

En particulier la composition de l'invention peut être appliquée à partir d'un dispositif aérosol.

De préférence, la composition selon l'invention se présente sous forme de gels, de crèmes ou de pâtes, de préférence, sous forme de crème.

La composition cosmétique selon l'invention peut être avantageusement utilisée pour le traitement cosmétique des cheveux. En particulier, la composition peut être employée pour le coiffage des cheveux, par exemple pour la mise en forme et/ou le maintien de la coiffure.

La présente invention concerne également un procédé de traitement cosmétique des cheveux, par exemple un procédé de mise en forme et/ou de maintien de la coiffure, qui consiste à appliquer sur les cheveux une quantité efficace d'une composition selon l'invention telle que décrite ci-avant, puis à effectuer un éventuel rinçage après un éventuel temps de pose.

De préférence, la composition selon l'invention n'est pas rincée.

Le procédé de l'invention peut se faire à température ambiante (25°C) ou sous chaleur à une température variant de 40 à 230 °C à l'aide de tout dispositif chauffant : casque, sèche cheveux, fer.

L'invention est illustrée plus en détails dans l'exemple suivant qui est présenté à titre illustratif et non limitatif de l'invention.

### EXEMPLE

On a réalisé deux crèmes de coiffage avec à partir des ingrédients indiqués en pourcentage en poids en produit en l'état dans le tableau ci-après :

| **Nom chimique** | **A** | **B** |
|---|---|---|
| Huile de vaseline⁽¹⁾ | 2 | 2,5 |
| Cire minérale d'hydrocarbures (C20/C60)⁽²⁾ | 15 | 13,5 |
| Copolymère vinylpyrrolidone / méthacrylate de di-méthyl aminoéthyl dans l'eau à 20%⁽³⁾ | 6 | - |
| Copolymère vinylpyrrolidone / acétate de vinyle (60/40) en solution aqueuse (50%) | 6 | 8 |
| Alcool stéarylique oxyéthyléné (2 OE)⁽⁴⁾ | 4 | 4 |
| Triéthanolamine (99%) | 1,2 | 1,2 |
| Mélange myristate/palmitate/stéarate de myristyle/cétyle/stéaryle ⁽⁵⁾ | 7 | 7 |
| Acide éthylène diamine tétracétique, sel disodique, 2 H₂O | 0,1 | 0,1 |
| Octane-1,2 diol | 1 | 1 |
| Acides gras d'origine végétale (Acide stéarique à 53% - acide palmitique - acide myristique)⁽⁶⁾ | 3 | 3 |
| Poly diméthylsiloxane (viscosité 5 Cst) | 4 | 7,5 |
| Cire microcristalline (point de fusion 74-79 °C)⁽⁷⁾ | 10 | 8,5 |
| Polymère carboxyvinylique ⁽⁸⁾ | 0,2 | 0,2 |
| Oleth-30⁽⁹⁾ | 6 | 6 |
| Parfum | 0,6 | 0,6 |
| Conservateur | 1 | 1 |
| Eau | QSP 100 | QSP 100 |

| | | |
|---|---|---|
| ⁽¹⁾BLANDOL commercialisé par la société SONNEBORN ⁽²⁾OZOKERITE WAX SP 1020 P commercialisé par la société STRAHL & PITSCH ⁽³⁾COPOLYMER 845-O commercialisé par la société Ashland ⁽⁴⁾BRIJ S2-SO-(SG) commercialisé par la société CRODA ⁽⁵⁾CRODAMOL MS-PA-(MH) commercialisé par la société CRODA ⁽⁶⁾PALMERA B1802CG commercialisé par la société KLK OLEO ⁽⁷⁾WHITE MICROCRYSTALLINE WAX SP-18 commercialisé par la société STRAHL & PITSCH ⁽⁸⁾SYNTHALEN K commercialisé par la société 3V ⁽⁹⁾EUMULGIN O 30 commercialisé par la société COGNIS | | |

On a appliqué les crèmes A et B sur cheveux secs.

Les crèmes obtenues sont faciles à prélever et à étaler dans les mains. On peut facilement les transférer depuis les mains vers les cheveux et elles sont faciles à répartir sur les cheveux. Les crèmes sont peu collantes mais permettent de mettre en forme la coiffure. En outre, la coiffure est rapide à mettre en forme.

On obtient une coiffure avec un rendu naturel. Les cheveux ne forment pas de paquets.

On obtient une bonne tenue de la coiffure, durable, et qui est en outre facile à recoiffer.

## Revendications

1. Composition cosmétique comprenant (i) au moins une cire minérale dans une teneur allant de 12 à 40 % en poids par rapport au poids total de la composition, (ii) au moins un acide gras comportant de 8 à 30 atomes de carbone, (iii) au moins une huile minérale, (iv) au moins un tensioactif (v) au moins un ester d'acide gras comprenant au moins 10 atomes de carbone et/ou d'alcool gras comprenant au moins 10 atomes de carbone et (vi) au moins un polymère fixant.

2. Composition selon la revendication précédente, dans laquelle la ou les cires minérales sont choisies parmi les cires microcristallines, l'ozokérite.

3. Composition selon l'une quelconque des revendications précédentes, **caractérisée en ce que** la ou les cires minérales sont présentes dans la composition dans des concentrations allant de 12 à 35% mieux de 12 à 30% en poids par rapport au poids total de la composition.

4. Composition selon l'une quelconque des revendications précédentes, dans laquelle le ou les acides gras sont choisis parmi l'acide stéarique, l'acide palmitique, l'acide myristique, l'acide béhénique et leurs mélanges.

5. Composition selon l'une quelconque des revendications précédentes dans laquelle le ou les acides gras sont présents dans la composition dans une teneur allant de 0,1 à 10%, de préférence de 0,5 à 7%, mieux de 1 à 5%, en poids par rapport au poids total de la composition.

6. Composition selon l'une quelconque des revendications précédentes, **caractérisée en ce que** l'huile ou les huiles minérales sont choisies parmi les mélanges d'huiles hydrocarbonées dérivées du pétrole, l'huile de paraffine, volatile ou non volatile, l'huile de vaseline, les polyoléfines et en particulier les polydécènes, les isoparaffines telles que l'isohexadécane, l'isododécane et les polyisobutylènes hydrogénés, et est de préférence l'huile de vaseline.

7. Composition selon l'une quelconque des revendications précédentes, **caractérisée en ce que** l'huile ou les huiles minérales sont présentes dans la composition dans une teneur allant de 0,1 à 10% en poids, et mieux encore de 0,5 à 7% en poids, et plus particulièrement de 1 à 5% en poids du poids total de la composition finale.

8. Composition selon l'une quelconque des revendications précédentes, dans laquelle le ou les tensioactifs sont choisis parmi les tensioactifs anioniques, cationiques, non ioniques, amphotères ou zwittérioniques ainsi que leurs mélanges, de préférence parmi les tensioactifs non ioniques.

9. Composition selon l'une quelconque des revendications précédentes, dans laquelle le ou les tensioactifs sont présents dans la composition dans une teneur allant de 0,1 à 30 % en poids, de préférence dans une teneur allant de 0,5 à 15% en poids, par rapport au poids total de la composition.

10. Composition selon l'une quelconque des revendications précédentes, **caractérisée en ce que** le ou les esters d'acide gras et/ou d'alcool gras sont solides à 25°C et à la pression atmosphérique (10 5 Pa).

11. Composition selon l'une quelconque des revendications précédentes, **caractérisée en ce que** le ou les esters d'acide gras et/ou d'alcool gras sont choisis parmi les esters d'acide gras saturé et de mono-alcool gras saturé.

12. Composition selon l'une quelconque des revendications précédentes, **caractérisée en ce que** les esters d'acide gras et/ou d'alcool gras sont choisis parmi les myristates de myristyle, de cétyle et de stéaryle, les palmitates de myristyle, de cétyle et de stéaryle, les stéarates de myristyle, de cétyle et de stéaryle, le béhénate de béhényle et leurs mélanges.

13. Composition selon l'une quelconque des revendications précédentes, **caractérisée en ce que** la concentration en ester d'acide gras et/ou d'alcool gras va de 1% à 20% en poids, de préférence de 2% à 15% en poids et plus préférentiellement de 4 % à 10 % en poids par rapport au poids total de la composition.

14. Composition selon l'une quelconque des revendications précédentes, **caractérisée en ce que** le ou les polymères fixants sont choisis parmi les polymères fixants non ioniques, anioniques, cationiques et amphotères, de préférence parmi les polymères fixants non ioniques et cationiques, mieux parmi les polymères fixants non ioniques.

15. Composition selon l'une quelconque des revendications précédentes, dans laquelle le ou les polymères fixants sont présents dans la composition dans des concentrations allant de 0,5 à 25%, de préférence de 0,7 à 20 %, de préférence encore de 1 à 17 % en poids par rapport au poids total de la composition.

16. Composition selon l'une quelconque des revendications précédentes **caractérisée en ce qu'**elle comprend un ou plusieurs alcools gras oxyéthylénés ayant de 1 à 5 motif(s) d'oxyde d'éthylène.

17. Composition selon l'une quelconque des revendications précédentes **caractérisée en ce qu'**elle comprend un ou plusieurs tensioactifs non ioniques oxyéthylénés à chaîne(s) grasse(s) insaturée(s) à au moins 10 motifs d'oxyde d'éthylène.

18. Composition selon l'une quelconque des revendications précédentes, qui comprend de l'eau, de préférence dans une teneur variant de 5 à 98%, de préférence de 10 à 95%, mieux de 20 à 80 %, et mieux encore de 30 à 70 % en poids par rapport au poids total de la composition.

19. Procédé de traitement cosmétique des fibres kératiniques, en particulier des fibres kératiniques humaines telles que les cheveux, **caractérisé en ce qu'**il consiste à appliquer sur lesdites fibres une quantité efficace d'une composition telle que définie selon l'une quelconque des revendications 1 à 18.

20. Utilisation d'une composition selon l'une quelconque des revendications 1 à 18 pour le traitement capillaire, notamment pour le traitement des fibres kératiniques et en particulier pour le maintien/la mise en forme des cheveux.

## Patentansprüche

1. Kosmetische Zusammensetzung, umfassend (i) mindestens ein Mineralwachs in einem Gehalt im Bereich von 12 bis 40 Gew.-%, bezogen auf das Gesamtgewicht der Zusammensetzung, (ii) mindestens eine Fettsäure mit 8 bis 30 Kohlenstoffatomen, (iii) mindestens ein Mineralöl, (iv) mindestens ein Tensid, (v) mindestens einen Ester einer Fettsäure mit mindestens 10 Kohlenstoffatomen und/oder eines Fettalkohols mit mindestens 10 Kohlenstoffatomen und (vi) mindestens ein fixierendes Polymer.

2. Zusammensetzung nach dem vorhergehenden Anspruch, wobei das Mineralwachs bzw. die Mineralwachse aus mikrokristallinen Wachsen und und Ozokerit ausgewählt ist bzw. sind.

3. Zusammensetzung nach einem der vorhergehenden Ansprüche, **dadurch gekennzeichnet, dass** das Mineralwachs bzw. die Mineralwachse in der Zusammensetzung in Konzentrationen im Bereich von 12 bis 35 Gew.-%, besser 12 bis 30 Gew.-%, bezogen auf das Gesamtgewicht der Zusammensetzung, vorliegt bzw. vorliegen.

4. Zusammensetzung nach einem der vorhergehenden Ansprüche, wobei die Fettsäure bzw. die Fettsäuren aus Stearinsäure, Palmitinsäure, Myristinsäure, Behensäure und Mischungen davon ausgewählt ist bzw. sind.

5. Zusammensetzung nach einem der vorhergehenden Ansprüche, wobei die Fettsäure bzw. die Fettsäuren in der Zusammensetzung in einem Gehalt im Bereich von 0,1 bis 10 Gew.-%, vorzugsweise 0,5 bis 7 Gew.-%, besser 1 bis 5 Gew.-%, bezogen auf das Gesamtgewicht der Zusammensetzung, vorliegt bzw. vorliegen.

6. Zusammensetzung nach einem der vorhergehenden Ansprüche, **dadurch gekennzeichnet, dass** das Mineralöl bzw. die Mineralöle aus Mischungen von Kohlenwasserstoffölen, die sich von Erdöl, flüchtigem oder nichtflüchtigem Paraffinöl, Vaselineöl, Polyolefinen und insbesondere Polydecenen, Isoparaffinen wie Isohexadecan oder Isododecan und hydrierten Polyisobutenen ausgewählt ist bzw. sind und vorzugsweise Vaselineöl ist.

7. Zusammensetzung nach einem der vorhergehenden Ansprüche, **dadurch gekennzeichnet, dass** das Mineralöl bzw. die Mineralöle in der Zusammensetzung an Gehalt im Bereich von 0,1 bis 10 Gew.-% und noch besser 0,5 bis 7 Gew.-% und spezieller 1 bis 5 Gew.-%, bezogen auf das Gesamtgewicht der fertigen Zusammensetzung, vorliegen.

8. Zusammensetzung nach einem der vorhergehenden Ansprüche, wobei das Tensid bzw. die Tenside aus anionischen, kationischen, nichtionischen, amphoteren oder zwitterionischen Tensiden sowie Mischungen davon, vorzugsweise aus nichtionischen Tensiden, ausgewählt ist bzw. sind.

9. Zusammensetzung nach einem der vorhergehenden Ansprüche, wobei das Tensid bzw. die Tenside in der Zusammensetzung in einem Gehalt im Bereich von 0,1 bis 30 Gew.-%, vorzugsweise in einem Gehalt von 0,5 bis 15 Gew.-%, bezogen auf das Gesamtgewicht der Zusammensetzung, vorliegt bzw. vorliegen.

10. Zusammensetzung nach einem der vorhergehenden Ansprüche, **dadurch gekennzeichnet, dass** der Ester bzw. die Ester einer Fettsäure und/oder eines Fettalkohols bei 25 °C und bei Normaldruck (10 5 Pa) fest ist bzw. sind.

11. Zusammensetzung nach einem der vorhergehenden Ansprüche, **dadurch gekennzeichnet, dass** der Ester bzw. die Ester einer Fettsäure und/oder eines Fettalkohols aus Estern einer gesättigten Fettsäure und eines gesättigten Monofettalkohols ausgewählt ist bzw. sind.

12. Zusammensetzung nach einem der vorhergehenden Ansprüche, **dadurch gekennzeichnet, dass** die Ester einer Fettsäure und/oder eines Fettalkohols aus Myristyl-, Cetyl- und Stearylmyristat, Myristyl-, Cetyl- und Stearylpalmitat, Myristyl-, Cetyl- und Stearylstearat, Behenylbehenat und Mischungen davon ausgewählt sind.

13. Zusammensetzung nach einem der vorhergehenden Ansprüche, **dadurch gekennzeichnet, dass** die Konzentration von Ester einer Fettsäure und/oder eines Fettalkohols im Bereich von 1 bis 20 Gew.-%, vorzugsweise von 2 bis 15 Gew.-% und spezieller von 4 bis 10 Gew.-%, bezogen auf das Gesamtgewicht der Zusammensetzung, liegt.

14. Zusammensetzung nach einem der vorhergehenden Ansprüche, **dadurch gekennzeichnet, dass** das fixierende Polymer bzw. die fixierenden Polymere aus nichtionischen, anionischen, kationischen und amphoteren fixierenden Polymeren, vorzugsweise aus nichtionischen und kationischen fixierenden Polymeren, besser aus nichtionischen fixierenden Polymeren, ausgewählt ist bzw. sind.

15. Zusammensetzung nach einem der vorhergehenden Ansprüche, wobei das fixierende Polymer bzw. die fixierenden Polymere in der Zusammensetzung in Konzentrationen im Bereich von 0,5 bis 25 Gew.-%, vorzugsweise von 0,7 bis 20 Gew.-%, weiter bevorzugt von 1 bis 17 Gew.-%, bezogen auf das Gesamtgewicht der Zusammensetzung, vorliegt bzw. vorliegen.

16. Zusammensetzung nach einem der vorhergehenden Ansprüche, **dadurch gekennzeichnet, dass** sie einen oder mehrere oxyethylenierte Fettalkohole mit 1 bis 5 Ethylenoxid-Einheiten umfasst.

17. Zusammensetzung nach einem der vorhergehenden Ansprüche, **dadurch gekennzeichnet, dass** sie ein oder mehrere oxyethylenierte nichtionische Tenside mit einer oder mehreren ungesättigten Fettketten mit mindestens 10 Ethylenoxid-Einheiten umfasst.

18. Zusammensetzen nach einem der vorhergehenden Ansprüche, die Wasser umfasst, vorzugsweise in einem Gehalt im Bereich von 5 bis 98 Gew.-%, vorzugsweise von 10 bis 95 Gew.-%, besser 20 bis 80 Gew.-% und noch besser 30 bis 70 Gew.-%, bezogen auf das Gesamtgewicht der Zusammensetzung.

19. Verfahren zur kosmetischen Behandlung von Keratinfasern, insbesondere menschlichen Keratinfasern wie dem Haar, **dadurch gekennzeichnet, dass** es daraus besteht, dass man auf die Fasern eine wirksame Menge einer Zusammensetzung gemäß einem der Ansprüche 1 bis 18 aufbringt.

20. Verwendung einer Zusammensetzung nach einem der Ansprüche 1 bis 18 zur Haarbehandlung, insbesondere zur Behandlung von Keratinfasern und insbesondere zur Formhaltung/Formgebung des Haars.

## Claims

1. Cosmetic composition comprising (i) at least one mineral wax in a content ranging from 12% to 40% by weight relative to the total weight of the composition, (ii) at least one fatty acid comprising from 8 to 30 carbon atoms, (iii) at least one mineral oil, (iv) at least one surfactant, (v) at least one ester of fatty acid comprising at least 10 carbon atoms and/or of fatty alcohol comprising at least 10 carbon atoms and (vi) at least one fixing polymer.

2. Composition according to the preceding claim, wherein the mineral wax(es) are chosen from microcrystalline waxes and ozokerite.

3. Composition according to either one of the preceding claims, **characterized in that** the mineral wax(es) are present in the composition in concentrations ranging from 12% to 35% and better still from 12% to 30% by weight relative to the total weight of the composition.

4. Composition according to any one of the preceding claims, wherein the fatty acid(s) are chosen from stearic acid, palmitic acid, myristic acid and behenic acid and mixtures thereof.

5. Composition according to any one of the preceding claims, wherein the fatty acid(s) are present in the composition in a content ranging from 0.1% to 10%, preferably from 0.5% to 7% and better still from 1% to 5% by weight relative to the total weight of the composition.

6. Composition according to any one of the preceding claims, **characterized in that** the mineral oil(s) are chosen from mixtures of hydrocarbon-based oils derived from petroleum, volatile or non-volatile liquid paraffin, liquid petroleum jelly, polyolefins and in particular polydecenes, isoparaffins such as isohexadecane or isododecane and hydrogenated polyisobutylenes, and is preferably liquid petroleum jelly.

7. Composition according to any one of the preceding claims, **characterized in that** the mineral oil(s) are present in the composition in a content ranging from 0.1% to 10% by weight and even better still from 0.5% to 7% by weight, and more particularly from 1% to 5% by weight of the total weight of the final composition.

8. Composition according to any one of the preceding claims, wherein the surfactant(s) are chosen from anionic, cationic, nonionic, amphoteric or zwitterionic surfactants and mixtures thereof, preferably from nonionic surfactants.

9. Composition according to any one of the preceding claims, wherein the surfactant(s) are present in the composition in a content ranging from 0.1% to 30% by weight, preferably in a content ranging from 0.5% to 15% by weight relative to the total weight of the composition.

10. Composition according to any one of the preceding claims, **characterized in that** the ester(s) of fatty acid and/or of fatty alcohol are solid at 25°C and at atmospheric pressure (10⁵ Pa).

11. Composition according to any one of the preceding claims, **characterized in that** the ester(s) of fatty acid and/or of fatty alcohol are chosen from esters of saturated fatty acid and of saturated fatty monoalcohol.

12. Composition according to any one of the preceding claims, **characterized in that** the the esters of fatty acid and/or of fatty alcohol are chosen from myristyl myristate, cetyl myristate, stearyl myristate, myristyl palmitate, cetyl palmitate, stearyl palmitate, myristyl stearate, cetyl stearate, stearyl stearate and behenyl behenate, and mixtures thereof.

13. Composition according to any one of the preceding claims, **characterized in that** the concentration of ester of fatty acid and/or of fatty alcohol ranges from 1% to 20% by weight, preferably from 2% to 15% by weight and more preferably from 4% to 10% by weight relative to the total weight of the composition.

14. Composition according to any one of the preceding claims, **characterized in that** the fixing polymer(s) are chosen from nonionic, anionic, cationic and amphoteric fixing polymers, preferably from nonionic and cationic fixing polymers, and better still from nonionic fixing polymers.

15. Composition according to any one of the preceding claims, wherein the fixing polymer(s) are present in the composition in concentrations ranging from 0.5% to 25%, preferably from 0.7% to 20% and more preferably from 1% to 17% by weight relative to the total weight of the composition.

16. Composition according to any one of the preceding claims, **characterized in that** it comprises one or more oxyethylenated fatty alcohols having from 1 to 5 ethylene oxide unit(s).

17. Composition according to any one of the preceding claims, **characterized in that** it comprises one or more oxyethylenated nonionic surfactants with unsaturated fatty chain(s) having at least 10 ethylene oxide units.

18. Composition according to any one of the preceding claims, which comprises water, preferably in a content ranging from 5% to 98%, preferably from 10% to 95%, better still from 20% to 80% and even better still from 30% to 70% by weight relative to the total weight of the composition.

19. Process for the cosmetic treatment of keratin fibers, in particular human keratin fibers such as the hair, **characterized in that** it consists in applying an effective amount of a composition, as defined in any one of Claims 1 to 18, to said fibers.

20. Use of a composition according to any one of Claims 1 to 18, for treating the hair, especially for treating keratin fibers and in particular for form retention/shaping of the hair.
